(19) Europäisches Patentamt
European Patent Office
Office européen des brevets

(11) EP 4 414 064 A1

(12) EUROPEAN PATENT APPLICATION
published in accordance with Art. 153(4) EPC

(43) Date of publication:
**14.08.2024 Bulletin 2024/33**

(21) Application number: **22878280.1**

(22) Date of filing: **09.09.2022**

(51) International Patent Classification (IPC):
***B01J 20/281*** (2006.01) ***B01J 20/291*** (2006.01)
***G01N 30/50*** (2006.01) ***G01N 30/56*** (2006.01)

(52) Cooperative Patent Classification (CPC):
**B01J 20/281; B01J 20/291; G01N 30/50; G01N 30/56**

(86) International application number:
**PCT/JP2022/033911**

(87) International publication number:
**WO 2023/058409 (13.04.2023 Gazette 2023/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.10.2021 JP 2021163997**

(71) Applicant: **JSR Corporation**
**Tokyo 105-8640 (JP)**

(72) Inventors:
- **AKIYAMA, Minato**
  **Tokyo 105-8640 (JP)**
- **KOBAYASHI, Kunihiko**
  **Tokyo 105-8640 (JP)**
- **INOUE, Yukiya**
  **Tokyo 105-8640 (JP)**
- **KIKUCHI, Masahiro**
  **Tokyo 105-8640 (JP)**

(74) Representative: **TBK**
**Bavariaring 4-6**
**80336 München (DE)**

# (54) METHOD FOR FILLING COLUMN WITH CHROMATOGRAPHY CARRIER, METHOD FOR STORING SLURRY, AND SLURRY

(57) Provided is a technique for suppressing a decrease in liquid permeability and pressure resistance characteristics during liquid passage of a chromatography carrier when a solvent is substituted with an aqueous solvent not containing a buffer.

A method for filling a column with a chromatography carrier, including Step 1 and Step 2 below:
(Step 1) a substitution step of substituting a slurry with an aqueous solvent not containing a buffer, in which the slurry contains a target substance-capturing chromatography carrier, a buffer having an acid dissociation constant (pKa) within a range of ± 1.0 of the isoelectric point of the carrier, and an aqueous solvent and has a pH of a liquid phase adjusted within a range of ± 2.0 of the isoelectric point of the carrier,
(Step 2) A filling step of filling a column with the slurry subjected to solvent substitution in Step 1

EP 4 414 064 A1

**Description**

Technical Field

The present invention relates to a method for filling a column with a chromatography carrier, a method for storing a slurry, and a slurry.

Background Art

In the field of biopharmaceuticals represented by antibody drugs and the like, a technology for expressing a target substance such as a protein has progressed. Accordingly, it is required by improving a productivity in a purification process.

In the purification process of antibody drugs, a purification method in which chromatography purifications of unit operations are combined has been developed into platforms. A purification in which an affinity ligand is bound to a carrier (affinity chromatography carrier) is most widely used as one unit operation (Patent Literature 1 to 3). Further, after purification using an affinity chromatography carrier, ion exchange chromatography, hydrophobic interaction chromatography, and the like are often performed in combination to remove impurities.

Citation List

Patent Literature

Patent Literature 1: JP 2020-2028 A
Patent Literature 2: WO 2015/199196 A
Patent Literature 3: WO 2017/090658 A

Summary of Invention

Technical Problem

Recently, it found that in a case where a column is filled with a chromatography carrier at a pH near an isoelectric point of the carrier, the desired liquid permeability is more easily acquired as a result of performing appropriate packing.

Therefore, in order to improve liquid permeability, the present inventors paid attention to incorporating a target substance-capturing chromatography carrier into a slurry while preliminarily adjusting a pH of a liquid phase of the slurry in the vicinity of an isoelectric point of a carrier. As a result of that the present inventors further studied, in the case of only adjusting a pH of a liquid phase of a slurry in the vicinity of an isoelectric point of a carrier, when a solvent is substituted with an aqueous solvent not containing a buffer for concentration adjustment, removal of an additive such as ethanol, removal of impurities, or the like, a pH is considerably increased, a liquid permeability is insufficient, and pressure resistance characteristics during liquid passage are also insufficient.

Therefore, the present invention is to provide a technique for suppressing a decrease in liquid permeability and pressure resistance characteristics during liquid passage of a chromatography carrier when a solvent is substituted with an aqueous solvent not containing a buffer.

Solution to Problem

The problem has been solved by the following means according to <1> to <9>.

<1> A method for filling a column with a chromatography carrier, including Step 1 and Step 2 below.

(Step 1) A substitution step of substituting a slurry with an aqueous solvent not containing a buffer, in which the slurry contains a target substance-capturing chromatography carrier, a buffer having an acid dissociation constant (pKa) within a range of $\pm$ 1.0 of the isoelectric point of the carrier and an aqueous solvent, and has a pH of a liquid phase adjusted within a range of $\pm$ 2.0 of the isoelectric point of the carrier,
(Step 2) A filling step of filling a column with the slurry subjected to solvent substitution in Step 1

<2> The method according to <1>, in which the pH of the liquid phase of the slurry subjected to solvent substitution

in Step 1 is in a range of 3 or more and 10 or less.

<3> The method according to <1> or <2>, in which an electrical conductivity of the liquid phase of the slurry subjected to solvent substitution in Step 1 is in a range of 0.006 mS/m or more and 200 mS/m or less.

<4> The method according to any one of <1> to <3>, in which the carrier is an affinity chromatography carrier or an ion exchange chromatography carrier.

<5> The method according to any one of <1> to <4>, in which the buffer is a buffer selected from a compound having a plurality of acidic groups in a molecule and a salt thereof.

<6> The method according to any one of <1> to <5>, in which a content of the buffer is 0.1 mM or more in the liquid phase of the slurry used in Step 1.

<7> A method for storing a slurry which is used by substituting with an aqueous solvent not containing a buffer before filling to a chromatography column, in which the slurry contains a target substance-capturing chromatography carrier, a buffer having an acid dissociation constant (pKa) within a range of ±1.0 of an isoelectric point of the carrier, and an aqueous solvent, and a pH of a liquid phase of the slurry is in a range of ±2.0 of the isoelectric point of the carrier.

<8> A slurry which is used by substituting with an aqueous solvent not containing a buffer before filling to a column, including: a target substance-capturing chromatography carrier; a buffer having an acid dissociation constant (pKa) within a range of ±1.0 of an isoelectric point of the carrier; and an aqueous solvent, in which a liquid phase pH is in a range of ±2.0 of the isoelectric point of the carrier.

<9> The slurry according to <8>, in which the buffer is a buffer selected from a compound having a plurality of acidic groups in a molecule and a salt thereof.

Advantageous Effects of Invention

According to the present invention, it is possible to suppress a decrease in liquid permeability and pressure resistance characteristics during liquid passage of a chromatography carrier when a solvent is substituted with an aqueous solvent not containing a buffer.

Description of Embodiments

<Method for Filling Column with Chromatography Carrier>

A filling method of the present invention is a method for filling a column with a chromatography carrier, including Step 1 and Step 2 below.

(Step 1) A substitution step of substituting a slurry with an aqueous solvent not containing a buffer, in which the slurry contains a target substance-capturing chromatography carrier, a buffer having an acid dissociation constant (pKa) within a range of ± 1.0 of the isoelectric point of the carrier, and an aqueous solvent and has a pH of a liquid phase adjusted within a range of ± 2.0 of the isoelectric point of the carrier,

(Step 2) A filling step of filling a column with the slurry subjected to solvent substitution in Step 1

((Step 1) Substitution Step)

Step 1 is a substitution step of substituting a slurry with an aqueous solvent not containing a buffer, in which the slurry contains a target substance-capturing chromatography carrier, a buffer having an acid dissociation constant (pKa) within a range of ± 1.0 of the isoelectric point of the carrier, and an aqueous solvent and has a pH of a liquid phase adjusted within a range of ± 2.0 of the isoelectric point of the carrier.

-Aqueous Solvent-

The slurry used in Step 1 contains an aqueous solvent.

Examples of the aqueous solvent include water and a mixed solution of water and a lower alcohol. Examples of the lower alcohol include one or more selected from ethanol and isopropanol, and ethanol is preferable for maintaining a storage state of a slurry.

A content of an aqueous solvent before substitution with an aqueous solvent not containing a buffer is preferably 20% by volume or more, more preferably 25% by volume or more, and particularly preferably 30% by volume or more in the slurry for improving stability, and is preferably 80% by volume or less, more preferably 70% by volume or less, and particularly preferably 60% by volume or less in the slurry in order to improve handleability. A specific range is preferably 20% by volume or more and 80% by volume or less, more preferably 25% by volume or more and 70% by volume or less, and particularly preferably 30% by volume or more and 60% by volume or less in the slurry. With such

a content, the storage stability is particularly improved.

The content of the aqueous solvent is calculated by subtracting a "content of a target substance-capturing chromatography carrier before substitution with an aqueous solvent not containing a buffer" which will be described later and a content of other components in the slurry, from a volume of the entire slurry.

In a case where a mixed solution of water and a lower alcohol is used as the aqueous solvent, a content proportion of the lower alcohol is preferably 5% by volume or more and 25% by volume or less in the mixed solution.

-Buffer-

The buffer contained in the slurry has an acid dissociation constant (pKa) within a range of ±1.0 of the isoelectric point of the target substance-capturing chromatography carrier ((isoelectric point - 1.0) ≤ pKa ≤ (isoelectric point + 1.0)), and when there are a plurality of acid dissociation constants (pKa), at least one may be within a range of ±1.0 of the isoelectric point of the carrier.

Here, in the present specification, the acid dissociation constant (pKa) is represented by a negative common logarithm pKa of an equilibrium constant Ka in a dissociation reaction in which hydrogen ions are released at 25°C and an ionic strength of 0.1 mol $dm^{-3}$. The pKa in the present invention is a value described in Chemistry Handbook, Basic Edition, revised Sixth Edition (Maruzen Publishing). In a substance not described therein, a manufacturer's published value may be adopted.

The acid dissociation constant (pKa) of the buffer is preferably in a range of (isoelectric point - 0.9) ≤ pKa ≤ (isoelectric point + 0.5), more preferably in a range of (isoelectric point - 0.8) ≤ pKa ≤ (isoelectric point ± 0), still more preferably in a range of (isoelectric point - 0.7) ≤ pKa ≤ (isoelectric point - 0.3), and particularly preferably in a range of (isoelectric point - 0.65) ≤ pKa ≤ (isoelectric point - 0.5), in order to improve liquid permeability and pressure resistance characteristics during liquid passage.

As the buffer, a buffer selected from a compound having a plurality of acidic groups (carboxy groups and the like) in a molecule and a salt thereof is preferable, a buffer selected from a compound having 2 to 5 acidic groups in a molecule and a salt thereof is more preferable, and a buffer selected from a compound having 3 acidic groups in a molecule and a salt thereof is particularly preferable in order to improve liquid permeability and pressure resistance characteristics during liquid passage.

In a case where the carrier is an affinity chromatography carrier (isoelectric point ≈ 5.0) having an immunoglobulin-binding protein as a ligand, a citrate buffer selected from citric acid (pKa1 = 2.90, pKa2 = 4.35, and pKa3 = 5.69) and salts thereof; a phthalate buffer selected from phthalic acid (pKa1 = 2.75 and pKa2 = 4.90) and salts thereof; a tartrate buffer selected from meso-tartaric acid (pKa1 = 2.95 and pKa2 = 4.46) and salts thereof; and an acetate buffer selected from acetic acid (pKa = 4.57) and salts thereof are preferred as the buffer. Among these, a citrate buffer, a phthalate buffer, and a tartrate buffer are preferable, and a citrate buffer is particularly preferable in order to improve liquid permeability and pressure resistance characteristics during liquid passage.

In a case where the carrier is a weakly acidic cation exchange chromatography carrier having an isoelectric point ≈ 9.0, a CHES (N-Cyclohexyl-2-amino ethanesulfonic acid) buffer selected from 2-(N-cyclohexylamino) ethanesulfonic acid (pKa = 9.5) and salts thereof; a borate buffer selected from boric acid (pKa = 8.98) and salts thereof; an ammonia buffer combining ammonia and an ammonium salt; and a tris buffer selected from trometamol (pKa = 8.2) and salts thereof are preferred as the buffer. Among these, a CHES buffer is preferable.

The values of the pKa described above are, except for CHES, values described in Chemistry Handbook, Basic Edition, revised Sixth Edition published by Maruzen Publishing Co., Ltd., and the value of CHES is a value described on the website of Merck Corporation (https://www.sigmaaldrich.com/JP/ja/product/mm/239779).

Examples of citrate, phthalate, tartrate, acetate, 2-(N-cyclohexylamino) ethanesulfonate, borate, and salts of trometamol include alkali metal salts such as sodium salts and potassium salts; and alkaline earth metal salts such as magnesium salts and calcium salts.

Here, a concentration of the buffer before substitution with an aqueous solvent not containing a buffer is preferably 0.1 mM or more in a slurry liquid phase. The concentration of the buffer is more preferably 1 mM or more, still more preferably 5 mM or more, still further preferably 10 mM or more, even more preferably 20 mM or more, and particularly preferably 30 mM or more in the slurry liquid phase, and is preferably 1000 mM or less, more preferably 750 mM or less, and particularly preferably 500 mM or less in the slurry liquid phase.

-Target Substance-Capturing Chromatography Carrier-

The slurry used in Step 1 contains a target substance-capturing chromatography carrier. Examples of a target substance include an antigen; an antibody such as a monoclonal antibody or a polyclonal antibody; a cell (a normal cell or a cancer cell such as a colorectal cancer cell or a circulating tumor cell); a nucleic acid such as DNA or RNA; and a biological substance such as a protein, a peptide, an amino acid, a sugar, a polysaccharide, a lipid, or a vitamin, and a

target substance may be a drug serving as a drug discovery target or a low molecular compound such as biotin.

The chromatography carrier may be any carrier as long as it has a target substance capturing property, and examples thereof include an affinity chromatography carrier, an ion exchange chromatography carrier, and a hydrophobic interaction chromatography carrier. Among these, the present invention is suitable for filling with an affinity chromatography carrier and an ion exchange chromatography carrier, and the present invention is particularly suitable for filling with an affinity chromatography carrier.

The isoelectric point of the carrier is not particularly limited, and is preferably 3.5 or more, more preferably 4 or more, still more preferably 4.5 or more, and particularly preferably 4.7 or more in order to suppress a pH change when performing substitution with an aqueous solvent. In addition, the isoelectric point is preferably 10 or less, and in a case where the target substance is an antibody or the like, the isoelectric point is more preferably 7 or less, still more preferably 6 or less, and particularly preferably 5.5 or less in order to efficiently capture the antibody or the like. A specific range is preferably 3.5 or more and 10 or less, more preferably 4 or more and 7 or less, still more preferably 4.5 or more and 6 or less, and particularly preferably 4.7 or more and 5.5 or less.

Here, in the present specification, the isoelectric point of the carrier is a pH value when a surface zeta potential measured by a flow potential method at 25°C becomes zero. At the time of measurement, a sample is disposed in a measurement cell such that a carrier to be measured is in contact with an electrolytic solution. The surface zeta potential is determined from the following calculation formula after an electrolytic solution is flowed into a measurement cell while changing a pressure and a flow potential at each pressure is measured. Specifically, measurement may be performed according to a method described in examples which will be described later.

$$\zeta = \frac{E}{\Delta P} \times \frac{\eta\lambda}{\varepsilon\varepsilon_0}$$

(Here, $\zeta$ represents a surface zeta potential, E represents a flow potential, $\Delta P$ represents a differential pressure between cells, $\eta$ represents a viscosity of an electrolytic solution, $\lambda$ represents a conductivity of an electrolytic solution, $\varepsilon$ represents a relative dielectric constant of an electrolytic solution, and $\varepsilon_0$ represents a dielectric constant in a vacuum.)

Examples of the affinity chromatography carrier include those which includes a ligand and a support and in which the ligand is immobilized on the support.

Examples of the ion exchange chromatography carrier include those having an ion exchange group in a molecule, and a cation exchange chromatography carrier is preferable, and a weakly acidic cation exchange chromatography carrier is more preferable.

Examples of a shape of the support and the ion exchange chromatography carrier include particles (beads), monoliths, plates, fibers, and membranes (including hollow fibers). Among these, particles are preferable, and porous particles are more preferable. In addition, the support and the ion exchange chromatography carrier are preferably water-insoluble.

Examples of the porous particles include organic porous particles, inorganic porous particles, organic-organic composite porous particles and organic-inorganic composite porous particles obtained by combining these, and organic porous particles are preferable, and porous particles containing a polymer are more preferable. Such porous particles may be natural polymer-based porous particles or synthetic polymer-based porous particles formed of polysaccharides such as agarose, dextran, and cellulose, and are preferably synthetic polymer-based porous particles in order to increase a dynamic binding capacity or improve the uniformity of particle diameters.

In a case where the carrier is an affinity chromatography carrier, the ligand may be a molecule that binds to a target substance, and examples thereof include a protein such as protein A, protein G, and avidin; a peptide such as insulin; a nucleic acid such as DNA and RNA; an enzyme; a chelate compound such as iminodiacetic acid; an antibody; an antigen; a hormone; carbohydrates such as heparin, Lewis X, and gangliosides; a receptor; an aptamer; a vitamin such as biotin and a derivative thereof; metal ions; and a synthetic dye, and further include a low molecular weight compound such as 2-aminophenylboric acid, 4-aminobenzamidine and glutathione. Note that the ligand exemplified above may be used in entirety thereof, and a fragment thereof obtained by recombinant, enzyme treatment, or the like may be used. In addition, an artificially synthesized peptide or a peptide derivative may be used.

Among the ligands, a protein, a peptide, a nucleic acid, an enzyme, and a chelate compound are preferable, a protein and a peptide are more preferable, and a protein is particularly preferable. In particular, among antibody affinity ligands of which a target substance is an antibody, an immunoglobulin-binding protein is preferable.

Examples of the antibody affinity ligand include a peptidic ligand, a proteinaceous ligand, and a chemically synthetic ligand (synthetic compound), and a peptidic or proteinaceous ligand is preferable. Among these, protein A, protein G, protein L, protein H, protein D, protein Arp, protein Fc$\gamma$R, an antibody-binding synthetic peptide ligand, and analog substances thereof are preferable, protein A, protein G, protein L, and analog substances thereof are more

preferable, and protein A and analog substances thereof are particularly preferable.

An immobilized amount of the ligand is preferably 10 mg or more and 300 mg or less, and more preferably 25 mg or more and 150 mg or less per 1 g of dry weight of the support, in order to increase the dynamic binding capacity.

In a case where the carrier is an ion exchange chromatography carrier, an ion exchange group is preferably a cation exchange group, and more preferably a weakly acidic cation exchange group. Specific examples thereof include one kind or two or more kinds selected from -SH, - $S^-M^+$, -$S^-$, -C(=O)OH, -C(=O)$O^-M^+$, -C(=O)$O^-$, -$S(=O)_2OH$, - $S(=O)_2O^-M^+$, -$S(=O)_2O^-$, -O-P(=O)$(OH)_2$, -O-P(=O)$(O^-M^+)_2$, -OP(=O)$(O^-)_2$, -O-P(=O)(OH)($O^-M^+$), and -O-P(=O)(OH)($O^-$) (each of the $M^+$ represents a counter ion). Examples of the counter ion represented by $M^+$ include alkali metal ions such as a sodium ion and a potassium ion; alkaline earth metal ions such as a magnesium ion and a calcium ion; an ammonium ion; and an organic ammonium ion.

Also, examples of the polymer contained in the synthetic polymer-based porous particles include polymers having a structural unit derived from a functional group-containing monomer. In a case where the carrier is an affinity chromatography carrier, a functional group contained in a monomer is preferably a group capable of immobilizing a ligand, and more preferably a group capable of immobilizing a ligand and capable of being used for an additional chemical reaction (such as a reaction with a crosslinking agent). Meanwhile, in a case where the carrier is an ion exchange chromatography carrier, a functional group contained in a monomer may be an ion exchange group or a functional group that can be used for a chemical reaction of a compound having an ion exchange group. For example, in a case where a structural unit derived from the functional group-containing monomer is a structural unit derived from the following cyclic ether group-containing monomer, an ion exchange group selected from -SH, -$S^-M^+$, and -$S^-$ can be introduced by reacting mercaptoalcohols such as thioglycerol.

Examples of the functional group contained in a monomer include a functional group selected from the group consisting of a cyclic ether group, a carboxy group, - C(=O)-O-C(=O)-, a succinimide oxycarbonyl group, a formyl group, a hydroxyl group, and an isocyanate group. Among these, a cyclic ether group is preferable.

Here, the "cyclic ether group" is preferably a cyclic ether group having 3 to 7 atoms that form a ring. The cyclic ether group may have an alkyl group as a substituent. Specific examples of the cyclic ether group include cyclic ether groups represented by the following Formulae (1) to (6), and a cyclic ether group represented by Formula (1), (3), or (6) is preferable, and a cyclic ether group represented by Formula (1) is more preferable.

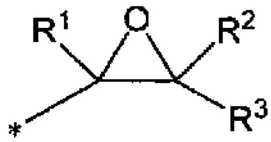

(1)

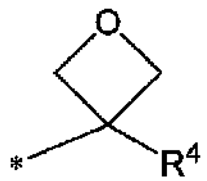

(2)

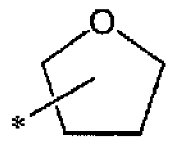

(3)

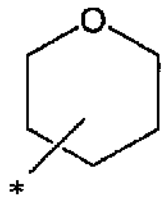

(4)

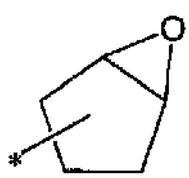

(5)

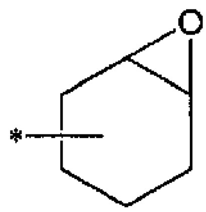

(6)

[In the formula, $R^1$ to $R^4$ each independently represent a hydrogen atom or an alkyl group, and * represents a bond.]

The number of carbon atoms of the alkyl group represented by $R^1$ to $R^4$ is preferably 1 to 4, and more preferably 1 or 2. The alkyl group may be linear or branched, and examples thereof include a methyl group, an ethyl group, an n-propyl group, an isopropyl group, an n-butyl group, a sec-butyl group, and a tert-butyl group. Also, $R^1$ to $R^4$ are preferably hydrogen atoms.

The functional group-containing monomer is preferably a monomer having a functional group capable of immobilizing a ligand and a polymerizable unsaturated group. Examples of such monomers include a (meth)acrylate-based

monomer having a cyclic ether group, such as glycidyl (meth)acrylate, 3-oxiranylpropyl (meth)acrylate, 4-oxiranylbutyl (meth)acrylate, 5-oxiranylpentyl (meth)acrylate, 6-oxiranylhexyl (meth)acrylate, 7-oxiranylheptyl (meth)acrylate, 8-oxiranyloctyl (meth)acrylate, (3-methyloxiranyl)methyl (meth)acrylate, 4-hydroxybutyl (meth)acrylate glycidyl ether, glycerin mono(meth)acrylate glycidyl ether, 3,4-epoxycyclohexylmethyl (meth)acrylate, 3,4-epoxycyclohexylethyl (meth)acrylate, 3,4-epoxycyclohexylpropyl (meth)acrylate, $\alpha$-(meth)acryl-$\omega$-glycidyl polyethylene glycol, and tetrahydrofurfuryl (meth)acrylate; an aromatic vinyl-based monomer having a cyclic ether group, such as (vinylbenzyl) glycidyl ether, (isopropenylbenzyl) glycidyl ether, (vinylphenethyl) glycidyl ether, (vinylphenylbutyl) glycidyl ether, (vinylbenzyloxyethyl) glycidyl ether, (vinylphenyl) glycidyl ether, (isopropenylphenyl) glycidyl ether, or 1,2-epoxy-3-(4-vinylbenzyl) propane; an allyl ether-based monomer having a cyclic ether group, such as allyl glycidyl ether; a (meth)acrylate-based monomer having an isocyanate group, such as isocyanatoethyl (meth)acrylate; and an unsaturated dicarboxylic anhydride-based monomer such as maleic anhydride, methyl maleic anhydride, and glutaconic anhydride, and further include (meth)acrylic acid, 3,4-epoxy-1-butene, and 3,4-epoxy-3-methyl-1-butene. These monomers can be used singly or in combination of two or more kinds thereof.

Among these monomers, a (meth)acrylate-based monomer having a cyclic ether group is preferable, and glycidyl (meth)acrylate is particularly preferable.

A content of the structural unit derived from the functional group-containing monomer is preferably 30% by mass or more, more preferably 50% by mass or more, and particularly preferably 70% by mass or more with respect to all structural units in the polymer, and is preferably 99% by mass or less, more preferably 95% by mass or less, and particularly preferably 90% by mass or less with respect to all structural units in the polymer.

In addition, as the polymer contained in the synthetic polymer-based porous particles, in addition to the structural unit derived from the functional group-containing monomer, a polymer further having a structural unit derived from a monomer (hereinafter, also referred to as other monomers) other than the functional group-containing monomer is preferable.

Examples of other monomers include a polymerizable unsaturated group-containing monomer having no functional group capable of immobilizing a ligand. Other monomers are roughly classified into non-crosslinkable monomers and crosslinkable monomers, and these monomers may be used alone or used in combination.

Examples of the non-crosslinkable monomers include a (meth)acrylate-based non-crosslinkable monomer, a (meth)acrylamide-based non-crosslinkable monomer, an aromatic vinyl-based non-crosslinkable monomer, a vinyl ketone-based non-crosslinkable monomer, a (meth)acrylonitrile-based non-crosslinkable monomer, and an N-vinylamide-based non-crosslinkable monomer. These can be used singly or in combination of two or more kinds thereof.

Examples of the (meth)acrylate-based non-crosslinkable monomer include methyl (meth)acrylate, ethyl (meth)acrylate, n-butyl (meth)acrylate, 4-tert-butyl (meth)acrylate, isobutyl (meth)acrylate, n-octyl (meth)acrylate, 2-ethylhexyl (meth)acrylate, cyclohexyl (meth)acrylate, methoxyethyl (meth)acrylate, hydroxyethyl (meth)acrylate, hydroxypropyl (meth)acrylate, glycerol mono(meth)acrylate, trimethylolethane mono(meth)acrylate, trimethylolpropane mono(meth)acrylate, butane triol mono(meth)acrylate, polyethylene glycol mono(meth)acrylate, methoxy polyethylene glycol (meth)acrylate, pentaerythritol mono(meth)acrylate, dipentaerythritol mono(meth)acrylate, and inositol mono(meth)acrylate. These can be used singly or in combination of two or more kinds thereof.

Also, examples of the (meth)acrylamide-based non-crosslinkable monomer include (meth)acrylamide, dimethyl (meth)acrylamide, hydroxyethyl (meth)acrylamide, (meth)acryloylmorpholine, and diacetone (meth)acrylamide. These can be used singly or in combination of two or more kinds thereof.

Also, examples of the aromatic vinyl-based non-crosslinkable monomer include styrenes such as styrene, $\alpha$-methylstyrene, halogenated styrene, 4-methylstyrene, 2,4-dimethylstyrene, 2,4,6-trimethylstyrene, ethylvinylbenzene, 4-isopropylstyrene, 4-n-butylstyrene, 4-isobutylstyrene, and 4-tert-butylstyrene; and vinylnaphthalenes such as 1-vinylnaphthalene and 2-vinylnaphthalene. These can be used singly or in combination of two or more kinds thereof.

Also, examples of the vinyl ketone-based non-crosslinkable monomer include ethyl vinyl ketone, propyl vinyl ketone, and isopropyl vinyl ketone. These can be used singly or in combination of two or more kinds thereof.

Also, examples of the (meth)acrylonitrile-based non-crosslinkable monomer include acrylonitrile and methacrylonitrile. These can be used singly or in combination of two or more kinds thereof.

Also, examples of the N-vinylamide-based non-crosslinkable monomer include N-vinylacetamide and N-vinylpropionamide. These can be used singly or in combination of two or more kinds thereof.

A content of the structural unit derived from the non-crosslinkable monomer is preferably 0% by mass or more, more preferably 0.01% by mass or more, still more preferably 0.1% by mass or more, and particularly preferably 0.2% by mass or more with respect to all structural units in the polymer, and is preferably 10% by mass or less, more preferably 5% by mass or less, and particularly preferably 3% by mass or less with respect to all structural units in the polymer.

In addition, examples of the crosslinkable monomers include a (meth)acrylate-based crosslinkable monomer, an aromatic vinyl-based crosslinkable monomer, and an allyl-based crosslinkable monomer. These can be used singly or in combination of two or more kinds thereof. In addition, as the crosslinkable monomer, a di- to penta- functional crosslinkable monomer is preferable, and a di- or trifunctional crosslinkable monomer is more preferable. Among the

crosslinkable monomers, a (meth)acrylate-based crosslinkable monomer and an aromatic vinyl-based crosslinkable monomer are preferable.

Examples of the (meth)acrylate-based crosslinkable monomer include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, tetrapropylene glycol di(meth)acrylate, polypropylene glycol di(meth)acrylate, 1,4-butanediol di(meth)acrylate, 1,6-hexanediol di(meth)acrylate, glycerin di(meth)acrylate, trimethylolethane di(meth)acrylate, trimethylolpropane di(meth)acrylate, trimethylolpropane tri(meth)acrylate, butane triol di(meth)acrylate, pentaerythritol di(meth)acrylate, pentaerythritol tri(meth)acrylate, pentaerythritol tetra(meth)acrylate, glucose di(meth)acrylate, glucose tri(meth)acrylate, glucose tetra(meth)acrylate, dipentaerythritol di(meth)acrylate, dipentaerythritol tri(meth)acrylate, dipentaerythritol tetra(meth)acrylate, dipentaerythritol penta(meth)acrylate, inositol di(meth)acrylate, inositol tri(meth)acrylate, inositol tetra(meth)acrylate, mannitol di(meth)acrylate, mannitol tri(meth)acrylate, mannitol tetra(meth)acrylate, and mannitol penta(meth)acrylate. These can be used singly or in combination of two or more kinds thereof.

Also, examples of the aromatic vinyl-based crosslinkable monomer include divinylbenzene, trivinylbenzene, divinyltoluene, divinylxylene, divinylethylbenzene, and divinylnaphthalene. These can be used singly or in combination of two or more kinds thereof.

Also, examples of the allyl-based crosslinkable monomer include diallyl phthalate, diallyl isophthalate, diallyl terephthalate, diallyl maleate, diallyl fumarate, diallyl itaconate, diallyl trimellitate, triallyl trimellitate, triallyl cyanurate, diallyl isocyanurate, and triallyl isocyanurate. These can be used singly or in combination of two or more kinds thereof.

Furthermore, in addition to those exemplified above, examples of the crosslinkable monomer can include a dehydration condensation reaction product of an amino alcohol such as diaminopropanol, trishydroxymethylaminomethane, or glucosamine with (meth)acrylic acid, and a conjugated diolefin such as butadiene or isoprene.

A content of the structural unit derived from the crosslinkable monomer is preferably 5% by mass or more, more preferably 10% by mass or more, and particularly preferably 15% by mass or more with respect to all structural units in the polymer, and is preferably 50% by mass or less, more preferably 40% by mass or less, and particularly preferably 30% by mass or less with respect to all structural units in the polymer.

In addition, in a case where the support and the ion exchange chromatography carrier are porous particles, the porous particles may be obtained by introducing a crosslinked structure or surface-modifying the crosslinked structure described in WO 2019/039545, JP 2011-252929 A, WO 2017/155105, JP 62-25102 A, or the like.

Examples of the crosslinking agent that provides a crosslinked structure include dicarboxylic acid dihydrazides such as oxalyl dihydrazide, malonic acid dihydrazide, succinic acid dihydrazide, 2,3-dihydroxysuccinic acid dihydrazide, glutaric acid dihydrazide, adipic acid dihydrazide, pimelic acid dihydrazide, octanedioic acid dihydrazide, nonanedioic acid dihydrazide, sebacic acid dihydrazide, dodecanedioic acid dihydrazide, phthalic acid dihydrazide, isophthalic acid dihydrazide, terephthalic acid dihydrazide, and quinolinic acid dihydrazide; tricarboxylic acid trihydrazides such as cyclohexanetricarboxylic acid trihydrazide; (alkylene bisimino)bis(oxoalkanoic acid) such as N1,N1-(ethane-1,2-diyl)bis(succinic acid monoamide); halohydrins such as epichlorohydrin, epibromohydrin, and dichlorohydrin; bisoxiranes such as resorcinol diglycidyl ether, neopentyl glycol diglycidyl ether, 1,6-hexanediol diglycidyl ether, hydrogenate bisphenol A diglycidyl ether, glycerol polyglycidyl ether, trimethylolpropane diglycidyl ether, diglycidyl terephthalate, diglycidyl orthophthalate, ethylene glycol diglycidyl ether, diethylene glycol diglycidyl ether, and propylene glycol diglycidyl ether; diamines such as 1,2-bis(2-aminoethoxy) ethane, m-xylylenediamine, 1,3-propanediamine, tetramethylenediamine, pentamethylenediamine, hexamethylenediamine, and 1,3-bis(aminomethyl) cyclohexane; and tri- or higher functional polyoxylanes. Residues of functional groups derived from the functional group-containing monomer are crosslinked by the crosslinking agent, via a partial structure derived from the crosslinking agent.

A content of the structural unit derived from the crosslinking agent is preferably 0.5% by mass or more, more preferably 1% by mass or more, and particularly preferably 5% by mass or more with respect to all structural units in the polymer, and is preferably 50% by mass or less, more preferably 40% by mass or less, and particularly preferably 35% by mass or less with respect to all structural units in the polymer.

In addition, in a case where the target substance-capturing chromatography carrier is a particulate carrier, a volume average particle diameter is preferably 40 to 150 um and more preferably 50 to 100 um. In addition, a coefficient of variation of the volume average particle diameter is preferably 40% or less, and more preferably 30% or less.

Also, a specific surface area of the target substance-capturing chromatography carrier is preferably 1 to 500 $m^2/g$, and more preferably 10 to 300 $m^2/g$.

In addition, a volume average pore size of the target substance-capturing chromatography carrier is preferably 10 to 300 nm.

The volume average particle diameter, the coefficient of variation, the specific surface area, and the volume average pore size can be measured by, for example, laser diffraction/scattering particle size distribution measurement.

The target substance-capturing chromatography carrier can be produced according to known methods described in WO 2015/119255 A, WO 2015/041218 A, US 6,399,750, Ljungquist C. et al., rEur. J. Biochem., 1989, Vol. 186, pp. 557-561, US 5,260,373, JP 2010-133733 A, JP 2010-133734 A, JP 2011-256176 A, and the like. For example, a mixed solution (monomer solution) containing a monomer composition and, if necessary, a porosifying agent is suspended in an aqueous medium and thermally polymerized in the presence of a polymerization initiator, the resulting porous particles are reacted with a crosslinking agent, and a ligand may be immobilized or an ion exchange group may be introduced into the resulting crosslinked porous particles. Note that the porous particle on which the ligand is immobilized may be brought into contact with a thiol compound such as methanethiol or thioglycerol to, for example, open a ring of an unreacted functional group. This processing may be performed with reference to the description of WO 2015/119255 A and the like. In addition, sieve classification may be performed before or after ligand immobilization or ion exchange group introduction.

A content of the target substance-capturing chromatography carrier before substitution with an aqueous solvent not containing a buffer is preferably 20% by volume or more, more preferably 30% by volume or more, and particularly preferably 40% by volume or more in the slurry, and is preferably 80% by volume or less, more preferably 75% by volume or less, and particularly preferably 70% by volume or less in the slurry. The specific range is preferably 20% by volume or more and 80% by volume or less, more preferably 30% by volume or more and 75% by volume or less, and particularly preferably 40% by volume or more and 70% by volume or less in the slurry. The % by volume of the carrier in the slurry can be calculated by filling a 250 mL volume glass measuring cylinder (according to JIS R3505 Class A) manufactured by Corning Incorporated with 200 mL of the slurry, and dividing a sedimentation volume after 3 hours of standing by a volume of the slurry with which a measuring cylinder is filled.

In addition, a content volume ratio of the target substance-capturing chromatography carrier to the aqueous solvent [(carrier)/(aqueous solvent)] is preferably 0.25 or more, more preferably 0.45 or more, and particularly preferably 0.65 or more, and is preferably 5.5 or less, more preferably 4 or less, and particularly preferably 3 or less. The specific range is preferably 0.25 or more and 5.5 or less, more preferably 0.45 or more and 4 or less, and particularly preferably 0.65 or more and 3 or less.

Also, the slurry may further contain a component other than the carrier, the buffer, and the aqueous solvent. Examples of such a component include a preservative.

-Slurry Liquid Phase pH (pH of Liquid Phase of Slurry before Substitution)-

The slurry used in Step 1 is obtained by adjusting the liquid phase pH of within the range of $\pm 2.0$ of the isoelectric point of the target substance-capturing chromatography carrier ((isoelectric point - 2.0) $\leq$ slurry liquid phase pH $\leq$ (isoelectric point + 2.0)). When adjusting the pH of the liquid phase of the slurry to be within the range of $\pm 2.0$ of the isoelectric point of the carrier, the liquid permeability and the pressure resistance characteristics during liquid passage of the chromatography carrier are hardly deteriorated even in a case where the solvent is substituted with an aqueous solvent not containing a buffer.

Here, in the present specification, the pH of the liquid phase of the slurry refers to a hydrogen ion index that can be measured at 25°C using a commercially available pH meter (for example, a tabletop pH meter manufactured by HORIBA, Ltd.).

The pH of the liquid phase of the slurry (pH of the liquid phase of the slurry before substitution) is preferably in a range of (isoelectric point - 1.5) $\leq$ slurry liquid phase pH $\leq$ (isoelectric point + 1.5), more preferably in a range of (isoelectric point - 1.0) $\leq$ slurry liquid phase pH $\leq$ (isoelectric point + 1.0), and particularly preferably in a range of (isoelectric point - 0.5) $\leq$ slurry liquid phase pH $\leq$ (isoelectric point + 0.5), in order to improve liquid permeability and pressure resistance characteristics during liquid passage.

In addition, the pH of the liquid phase of the slurry is preferably 3 or more, more preferably 3.5 or more, and particularly preferably 4 or more in order to improve liquid permeability and pressure resistance characteristics during liquid passage, and is preferably 10 or less, more preferably 7 or less, and particularly preferably 6 or less in order to improve liquid permeability and pressure resistance characteristics during liquid passage. A specific range is preferably 3 or more and 10 or less, more preferably 3.5 or more and 7 or less, and particularly preferably 4 or more and 6 or less.

-Substitution-

Step 1 is a substitution step of substituting the slurry with an aqueous solvent not containing a buffer. Here, in the present specification, the "aqueous solvent not containing a buffer" includes not only an aqueous solvent having a buffer concentration of 0 mM but also an aqueous solvent containing a trace amount of a buffer or a salt, and may be an aqueous solvent having a concentration of one or more selected from the buffer and the salt of 0 to 1 mM, and an aqueous solvent having a buffer concentration of 0 to 0.01 mM is preferable, and an aqueous solvent having a buffer concentration of 0 mM is more preferable. In a case where such an aqueous solvent not containing a buffer is used, it is not necessary to perform a step of dissolving a buffer or a salt or a step of adjusting a pH before performing filling,

which is simple, and equipment corrosion or contamination due to precipitation of salts can be suppressed. According to Step 1, a solvent of the slurry is substituted with an aqueous solvent not containing a buffer. In the filling method of the present invention, by including Step 1, adjusting the concentration of the slurry can be easily performed, and removing additives such as ethanol and impurities in the slurry can be performed, so that convection and the like that can occur in the column can be suppressed, and appropriate packing is performed.

Examples of the aqueous solvent not containing a buffer include water and a mixed solution of water and a lower alcohol. Examples of the lower alcohol include one or more selected from ethanol and isopropanol. In a case where a mixed solution of water and a lower alcohol is used as the aqueous solvent not containing a buffer, a content proportion of the lower alcohol is preferably 5% by volume or more and 25% by volume or less in the mixed solution.

A substitution operation in Step 1 may be performed in the same manner as the conventional solvent substitution except that the slurry is used. For example, removing a solvent by a natural sedimentation method, a centrifugal separation method, filtration, or the like and adding an aqueous solvent not containing a buffer may be performed in combination, and a substitution operation using a natural sedimentation method is preferable.

In the case of the substitution operation using the natural sedimentation method, Step 1 preferably includes a sedimentation step of precipitating a carrier in a slurry, a removal step of removing 50% by volume or more and 95% by volume or less of the slurry component other than the carrier after the sedimentation step, and an aqueous solvent addition step of adding an aqueous solvent not containing a buffer in a volume amount of 70% or more and 150% or less as compared with a volume of slurry components other than the carrier removed in the removal step, and more preferably includes a step of repeating a combination of the sedimentation step, the removal step, and the aqueous solvent addition step twice or more and 10 times or less. By including such a step, it is possible to suppress variations in physical properties of the slurry at the time of substitution, and it is possible to obtain a slurry in which appropriate packing is easily performed.

The pH of the liquid phase of the slurry after solvent substitution in Step 1 is preferably 3 or more, more preferably 3.5 or more, still more preferably 4 or more, and particularly preferably 4.5 or more in order to improve liquid permeability and pressure resistance characteristics during liquid passage, and is preferably 10 or less, more preferably 7 or less, and particularly preferably 6 or less in order to improve liquid permeability and pressure resistance characteristics during liquid passage. The specific range is preferably 3 or more and 10 or less, more preferably 3.5 or more and 7 or less, still more preferably 4 or more and 6 or less, and particularly preferably 4.5 or more and 6 or less.

The pH of the liquid phase of the slurry after substitution refers to a hydrogen ion index that can be measured at 25°C using a commercially available pH meter (for example, a tabletop pH meter manufactured by HORIBA, Ltd.) in the same manner as for the pH of the liquid phase of the slurry before substitution.

An electrical conductivity of the liquid phase of the slurry after solvent substitution in Step 1 is preferably 0.006 mS/m or more, more preferably 0.01 mS/m or more, still more preferably 0.1 mS/m or more, and particularly preferably 0.5 mS/m or more in order to improve liquid permeability and pressure resistance characteristics during liquid passage, and is preferably 200 mS/m or less, more preferably 150 mS/m or less, still more preferably 100 mS/m or less, still more preferably 50 mS/m or less, and particularly preferably 20 mS/m or less in order to suppress convection that can occur in the column and to perform packing appropriately. The specific range is preferably 0.006 mS/m or more and 200 mS/m or less, more preferably 0.01 mS/m or more and 150 mS/m or less, still more preferably 0.01 mS/m or more and 100 mS/m or less, still further preferably 0.1 mS/m or more and 50 mS/m or less, and particularly preferably 0.5 mS/m or more and 20 mS/m or less.

The electrical conductivity of the liquid phase of the slurry after substitution can be measured in accordance with JIS K0130 (General Rules for Electrical Conductivity Measurement Methods) using an electrical conductivity meter (for example, D-74SE manufactured by HORIBA, Ltd.).

((Step 2) Filling Step)

Step 2 is a filling step of filling a column with the slurry subjected to solvent substitution in Step 1.

The filling operation in Step 2 may be performed in the same manner as in the conventional filling except that the slurry subjected to solvent substitution in Step 1 is used. For example, it can be performed with reference to the description of the packing protocol or the like of Ampshere A3 manufactured by JSR Corporation.

Further, according to the filling method of the present invention, it is possible to provide a technique for suppressing a decrease in liquid permeability and pressure resistance characteristics during liquid passage of a chromatography carrier when a solvent is substituted with an aqueous solvent not containing a buffer. The filling method of the present invention is suitable for filling with an affinity chromatography carrier and an ion exchange chromatography carrier, and particularly suitable for filling with an affinity chromatography carrier.

<Method for Storing Slurry and Slurry>

A method for storing a slurry according to the present invention is a method for storing a slurry which is used by substituting with an aqueous solvent not containing a buffer before filling to a chromatography column, in which the slurry contains a target substance-capturing chromatography carrier, a buffer having an acid dissociation constant (pKa) within a range of ±1.0 of an isoelectric point of the carrier and an aqueous solvent, and a pH of a liquid phase of the slurry is in a range of ±2.0 of the isoelectric point of the carrier.

A slurry which is used by substituting with an aqueous solvent not containing a buffer before filling to a column according to the present invention includes a target substance-capturing chromatography carrier; a buffer having an acid dissociation constant (pKa) within a range of ±1.0 of an isoelectric point of the carrier; and an aqueous solvent, in which a pH of a liquid phase is in a range of ±2.0 of the isoelectric point of the carrier.

A method for storing a slurry according to the present invention may be performed in the same manner as a conventional method, except that the slurry containing a target substance-capturing chromatography carrier, a buffer having an acid dissociation constant (pKa) within a range of ±1.0 of an isoelectric point of the carrier and an aqueous solvent, and having a pH of a liquid phase of the slurry being in a range of ±2.0 of the isoelectric point of the carrier, and being used by being substituting with an aqueous solvent not containing a buffer is stored.

The slurry of the present invention can be used for purification of a target substance. The purification of the target substance may be performed according to a conventional method.

The meaning of various wordings, a content of each component, a ratio thereof, and the like in the method for storing a slurry and the slurry of the present invention are the same as the meaning of various wordings, a content of each component, a ratio thereof, and the like described in the filling method of the present invention.

[Examples]

Hereinafter, the present invention will be described in detail with reference to Examples, but the present invention is not limited to these Examples.

(Example 1 Synthetic Polymer-based Affinity Chromatography Carrier Slurry)

(1) 2.69 g of polyvinyl alcohol (PVA-217 manufactured by Kuraray Co., Ltd.) was added to 448 g of pure water, and heated and stirred to dissolve the polyvinyl alcohol, thereby preparing an aqueous solution S. Meanwhile, a monomer composition composed of 3.99 g of divinylbenzene (manufactured by Wako Pure Chemical Industries, Ltd.) and 14.15 g of glycidyl methacrylate (manufactured by Mitsubishi Gas Chemical Company, Inc.) was dissolved in 29.38 g of 2-octanone (manufactured by Toyo Gosei Co., Ltd.) to prepare a monomer solution. Subsequently, an entire amount of the aqueous solution S was charged into a separable flask, a thermometer, a stirring blade, and a cooling tube were attached, the flask was set in a hot water bath, and stirring was started under a nitrogen atmosphere. The entire amount of the monomer solution was charged into the separable flask, and heated with a warm water bath, and 1.34 g of 2,2'-azobis(methyl isobutyrate) (manufactured by Wako Pure Chemical Industries, Ltd.) was added thereto when an internal temperature reached 85°C, and then the internal temperature was set to 86°C.

(2) Thereafter, stirring was performed for 3 hours while maintaining the temperature at 86°C. Subsequently, a reaction solution was cooled, and then the reaction solution was filtered and washed with pure water and ethanol. The washed particles were dispersed in pure water, and decantation was performed three times to remove small particles. Next, the particles were dispersed in pure water so that a concentration of the particles was 10% by mass to obtain a porous particle dispersion. The porous particles contained in this dispersion are referred to as "porous solid phase support 1".

(3) Thereafter, 0.956 g of adipic acid dihydrazide (manufactured by Tokyo Chemical Industry Co., Ltd.) and 1.418 g of diisopropylethylamine (manufactured by Tokyo Chemical Industry Co., Ltd.) were added to 100 g of a porous solid phase support 1 dispersion, a mixture was heated to 70°C, and stirred for 8 hours while maintaining the temperature at 70°C. Subsequently, a reaction solution was cooled, and then the reaction solution was filtered and washed with pure water and ethanol. The obtained particles were recovered with pure water to obtain a porous particle dispersion. The porous particles contained in this dispersion are referred to as "porous solid phase support 2".

(4) Thereafter, the porous solid phase support 2 was put into a SATO-type vibration sieve machine (400DB-2S manufactured by Koei Sangyo Co., Ltd.) equipped with a wire mesh (manufactured by Taiyo Wire Netting Co., Ltd.) having a mesh size of 77 um, and the sieve passed components (liquid that passed through the sieve after wet classification) were recovered. Next, the components passed through the sieve were put into the SATO-type vibration sieve machine in which the wire mesh was changed to a wire mesh having a mesh size of 32 um (manufactured by

Taiyo Wire Netting Co., Ltd.), and the sieve residue (porous particles remaining on the sieve after wet classification) was recovered with pure water to obtain a porous particle dispersion. The porous particles contained in this dispersion are referred to as "porous solid phase support 3".

(5) Next, 0.15 g of modified protein A (rSPA manufactured by Repligen) was dissolved in 40 mL of 1.2 M sodium sulfate/0.1 M sodium phosphate buffer (pH 6.6) to obtain a protein A solution, and the porous solid phase support 3 (corresponding to 1 g in terms of particle dry mass) was added to the protein A solution. The dispersion was shaken and stirred at 25°C for 10 hours to immobilize protein A on the particles.

Next, the produced protein A-immobilized particles were dispersed in 40 mL of 1.0 M α-thioglycerol (manufactured by Tokyo Chemical Industry Co., Ltd.)/0.1 M sodium sulfate (pH 8.3), and shaken and stirred at 25°C for 17 hours to ring-open the unreacted epoxy group. Furthermore, the protein A-immobilized particles obtained by ring-opening the unreacted epoxy group were washed with a 0.1 M sodium phosphate buffer (pH 6.6), a 0.1 M sodium hydroxide aqueous solution, a 0.1 M sodium citrate buffer (pH 3.2), and a 0.1 M sodium phosphate buffer (pH 7.5) to obtain a ligand-immobilizing carrier V1. The obtained carrier was substituted with a 0.1 M sodium citrate buffer (pH 5.0) prepared using a 16% by volume aqueous ethanol solution to obtain a ligand-immobilizing carrier slurry W1 (solid content: 50% by volume).

(Example 2 Synthetic Polymer-based Affinity Chromatography Carrier Slurry)

A ligand-immobilizing carrier slurry W2 was obtained in the same manner as in Example 1 except that in Step (5) of Example 1, the 0.1 M sodium citrate buffer (pH 5.0) prepared using a 16% by volume aqueous ethanol solution was changed to a 0.1 M sodium citrate buffer (pH 3.5) prepared using a 16% by volume aqueous ethanol solution.

(Example 3 Synthetic Polymer-based Affinity Chromatography Carrier Slurry)

A ligand-immobilizing carrier slurry W3 was obtained in the same manner as in Example 1 except that in Step (5) of Example 1, the 0.1 M sodium citrate buffer (pH 5.0) prepared using a 16% by volume aqueous ethanol solution was changed to a 0.1 M sodium citrate buffer (pH 6.5) prepared using a 16% by volume aqueous ethanol solution.

(Example 4 Synthetic Polymer-based Affinity Chromatography Carrier Slurry)

A ligand-immobilizing carrier slurry W4 was obtained in the same manner as in Example 1 except that in Step (5) of Example 1, the 0.1 M sodium citrate buffer (pH 5.0) prepared using a 16% by volume aqueous ethanol solution was changed to a 0.1 M sodium citrate buffer (pH 5.0).

(Example 5 Synthetic Polymer-based Affinity Chromatography Carrier Slurry)

A ligand-immobilizing carrier slurry W5 was obtained in the same manner as in Example 1 except that in Step (5) of Example 1, the 0.1 M sodium citrate buffer (pH 5.0) prepared using a 16% by volume aqueous ethanol solution was changed to a 0.1 M sodium acetate buffer (pH 5.0) prepared using a 16% by volume aqueous ethanol solution.

(Example 6 Synthetic Polymer-based Affinity Chromatography Carrier Slurry)

A ligand-immobilizing carrier slurry W6 was obtained in the same manner as in Example 1 except that in Step (5) of Example 1, the 0.1 M sodium citrate buffer (pH 5.0) prepared using a 16% by volume aqueous ethanol solution was changed to a 0.1 M sodium acetate buffer (pH 3.5) prepared using a 16% by volume aqueous ethanol solution.

(Example 7 Agarose-based Affinity Chromatography Carrier Slurry)

Mabselect SuRe (manufactured by Cytiva) was prepared, and a dispersion medium thereof was substituted with a 0.1 M sodium citrate buffer (pH 5.0) prepared using a 16% by volume aqueous ethanol solution to obtain a ligand-immobilizing carrier slurry W7 (solid content: 50% by volume).

(Example 8 Agarose-based Affinity Chromatography Carrier Slurry)

Mabselect SuRe (manufactured by Cytiva) was prepared, and a dispersion medium thereof was substituted with a 0.1 M sodium acetate buffer (pH 5.0) prepared using a 16% by volume aqueous ethanol solution to obtain a ligand-immobilizing carrier slurry W8 (solid content: 50% by volume).

(Example 9 Ion Exchange Chromatography Carrier Slurry)

0.837 g of 1,2-bis(2-aminoethoxy)ethane (manufactured by Tokyo Chemical Industry Co., Ltd.) and 0.452 g of diisopropylethylamine (manufactured by Tokyo Chemical Industry Co., Ltd.) were added to 100 g of 10% by mass aqueous dispersion of the porous solid phase support 1 obtained in Example 1, a mixture was heated to 70°C, and stirred for 8 hours while maintaining the temperature at 70°C. Then, 12.215 g of $\alpha$-thioglycerol (manufactured by Tokyo Chemical Industry Co., Ltd.) was added thereto, and the mixture was stirred at 70°C for 3 hours. Subsequently, a reaction solution was cooled, and then the reaction solution was filtered and washed with pure water and ethanol. The obtained particles were recovered with pure water to obtain a weak ion exchange ligand-immobilizing carrier dispersion. The carrier contained in this dispersion is referred to as a "ligand-immobilizing carrier V2".

Thereafter, the ligand-immobilizing carrier V2 was classified and recovered in the same manner as in (4) in Example 1, and the recovered carrier was substituted with 0.1 M CHES (N-Cyclohexyl-2-aminoethanesulfonic acid) buffer (pH 9.0) prepared using a 16% by volume aqueous ethanol solution to obtain a ligand-immobilizing carrier slurry W9 (solid content: 50% by volume).

(Example 10 Synthetic Polymer-based Affinity Chromatography Carrier Slurry)

A ligand-immobilizing carrier slurry W10 was obtained in the same manner as in Example 1 except that in Step (5) of Example 1, the 0.1 M sodium citrate buffer (pH 5.0) prepared using a 16% by volume aqueous ethanol solution was changed to a 1 mM sodium citrate buffer (pH 5.0) prepared using a 16% by volume aqueous ethanol solution.

(Comparative Example 1 Synthetic Polymer-based Affinity Chromatography Carrier Slurry)

A ligand-immobilizing carrier slurry Y1 was obtained in the same manner as in Example 1 except that in Step (5) of Example 1, the 0.1 M sodium citrate buffer (pH 5.0) prepared using a 16% by volume aqueous ethanol solution was changed to a 0.1 M sodium phosphate buffer (pH 6.5) prepared using a 16% by volume aqueous ethanol solution.

(Comparative Example 2 Synthetic Polymer-based Affinity Chromatography Carrier Slurry)

A ligand-immobilizing carrier slurry Y2 was obtained in the same manner as in Example 1 except that in Step (5) of Example 1, the 0.1 M sodium citrate buffer (pH 5.0) prepared using a 16% by volume aqueous ethanol solution was changed to a 0.1 M sodium phosphate buffer (pH 7.5) prepared using a 16% by volume aqueous ethanol solution.

(Comparative Example 3 Ion Exchange Chromatography Carrier Slurry)

A ligand-immobilizing carrier slurry Y3 was obtained in the same manner as in Example 9 except that in Example 9, the 0.1 M CHES buffer (pH 9.0) prepared using a 16% by volume aqueous ethanol solution was changed to a 0.1 M sodium acetate buffer (pH 5.0) prepared using a 16% by volume aqueous ethanol solution.

(Test Example 1 Measurement of Isoelectric Point of Carrier)

An isoelectric point of the carrier in each of the slurries W1 to W10 and Y1 to Y3 obtained in each of Examples and Comparative Examples was measured with a zeta potential analyzer for solid surface analysis (SurPASS manufactured by Anton Paar). That is, a cylinder cell was filled with 0.5 mL of the carrier, and equilibrated with a 10 mM potassium chloride aqueous solution of which a pH was adjusted to 11 with sodium hydroxide, and then a zeta potential was measured by the principle of a flow potential method while changing the pH with 0.05 M hydrochloric acid. The pH at which the zeta potential reached 0 mV was recorded as the isoelectric point of the carrier. The results are shown in Tables 1 to 3.

(Test Example 2: Evaluation of Liquid Permeability)

For the slurries W1 to W10 and Y1 to Y3 obtained in Examples and Comparative Examples, precipitating the carrier, discarding a supernatant of 90% by volume of the slurry components other than the carrier, and adding an equal amount of pure water were repeated five times to substitute a dispersion medium, and a pH and electrical conductivity of the resulting substituted slurry were recorded. Also, for the slurry W1, precipitating the carrier, discarding a supernatant of 90% by volume of the slurry components other than the carrier, and adding an equal amount of pure water were repeated twice to substitute a dispersion medium, and a pH and electrical conductivity of the resulting substituted slurry were recorded for Example 11. Also, for the slurry W1, precipitating the carrier, discarding a supernatant of 90% by

volume of the slurry components other than the carrier, and adding an equal amount of pure water were repeated eight times to substitute a dispersion medium, and a pH and electrical conductivity of the resulting substituted slurry were recorded for Example 12. The results are shown in Tables 1 to 3. The electrical conductivity was measured in accordance with JIS K0130 using D-74SE manufactured by HORIBA, Ltd.

Two Hiscale 26/20 columns connected to have an inner diameter of 26 mm and a filling height of 200 mm (manufactured by Cytiva) were filled with the substituted slurry, and the columns were connected to AKTA AVANT 150 manufactured by Cytiva. Next, after passing pure water at a linear flow rate of 100 cm/hr for 30 minutes, an upper column was removed, and the carrier was packed in the column by adhering an adapter to a carrier layer. Thereafter, a linear flow rate was adjusted until a back pressure of 0.25 MPa was generated, and the linear flow rate at that time was recorded. The results are shown in Tables 1 to 3. It can be said that the larger the value of the linear flow rate, the better the liquid permeability.

(Test Example 3 Evaluation of Pressure Resistance Characteristics during Liquid Passage)

For the slurries W1 to W10 and Y1 to Y3 obtained in Examples and Comparative Examples, dispersion medium substitution and packing were performed in the same manner as in Test Example 2, then pure water was passed at a linear flow rate of 800 cm/hr, and a back pressure and a compression ratio (change rate of filling height) at that time were recorded. The compression ratio was calculated using the following equation. The results are shown in Tables 1 to 3. It can be said that the smaller the values of the back pressure and the compression ratio, the better the pressure resistance characteristics.

(Compression ratio) = 1 - {(Filling height after passing liquid at 800 cm/h)/(Filling height before passing liquid at 800 cm/h)}

(Test Example 4 Evaluation of Dynamic Binding Capacity)

A DBC of the carrier in the slurries W1 to W8, W10, and Y1 to Y3 with respect to a protein (Human IgG antibody, HGG-1000 manufactured by Equitech Bio Inc.) at a linear flow rate of 300 cm/hr was measured using AKTAprime plus manufactured by GE Healthcare. After performing the dispersion medium substitution in the same manner as in Test Example 2, a column container having a volume of 4 mL (5 mmφ × 200 mm in length) was used, and a protein obtained by dissolving 5 mg/mL of protein in a 20 mM sodium phosphate/150 mM sodium chloride aqueous solution (pH 7.5) was used, and the DBC was determined from the amount of captured protein at 10% breakthrough at an elution tip and a column packing volume. The results are shown in Tables 1 to 3.

[Table 1]

| | | Example | | | | | | | | |
|---|---|---|---|---|---|---|---|---|---|---|
| | | 1 | 2 | 3 | 4 | 5 | 6 | 7 | 8 | 9 |
| Kind of carrier | Synthetic polymer-based affinity carrier | ○ | ○ | ○ | ○ | ○ | ○ | | | |
| | Agarose-based affinity carrier | | | | | | | ○ | ○ | |
| | Ion exchange carrier | | | | | | | | | ○ |
| Slurry liquid phase | Kind of salt | Citrate | Citrate | Citrate | Citrate | Acetate | Acetate | Citrate | Acetate | CHES |
| | pKa of buffer (25°C) | pKa1=2.90<br>pKa2=4.35<br>pKa3=5.69 | pKa1=2.90<br>pKa2=4.35<br>pKa3=5.69 | pKa1=2.90<br>pKa2=4.35<br>pKa3=5.69 | pKa1=2.90<br>pKa2=4.35<br>pKa3=5.69 | pKa=4.57 | pKa=4.57 | pKa1=2.90<br>pKa2=4.35<br>pKa3=5.69 | pKa=4.57 | pKa=9.5 |
| | (pKa of buffer) - (Isoelectric point of carrier) | -0.65<br>(pKa2) | -0.65<br>(pKa2) | -0.65<br>(pKa2) | -0.65<br>(pKa2) | -0.43 | -0.43 | -0.65<br>(pKa2) | -0.43 | +0.5 |
| | Slurry liquid phase pH (25°C) | 5.0 | 3.5 | 6.5 | 5.0 | 5.0 | 3.5 | 5.0 | 5.0 | 9.0 |
| | (Slurry liquid phase pH) - (Isoelectric point of carrier) | ±0 | -1.5 | +1.5 | ±0 | ±0 | -1.5 | ±0 | ±0 | ±0 |
| | Liquid composition | Water + EtOH | Water + EtOH | Water + EtOH | Water | Water + EtOH | Water + EtOH | Water + EtOH | Water + EtOH | Water + EtOH |
| Characteristics | Isoelectric point of carrier PI (25°C) | 5.0 | 5.0 | 5.0 | 5.0 | 5.0 | 5.0 | 5.0 | 5.0 | 9.0 |
| | pH after substitution (25°C) | 5.0 | 4.0 | 6.5 | 5.0 | 5.0 | 4.0 | 5.0 | 5.0 | 9.0 |
| | Electrical conductivity after substitution mS/m (25°C) | 25.2 | 12.8 | 30.4 | 28.3 | 7.8 | 4.2 | 24.7 | 8.0 | 13.7 |
| Evaluation | Liquid permeability (cm/h) @0.25 MPa | 850 | 820 | 750 | 850 | 830 | 810 | 1450 | 1450 | 1000 |
| | Back pressure (MPa) @800 cm/hr | 0.21 | 0.23 | 0.28 | 0.21 | 0.23 | 0.24 | 0.1 | 0.1 | 0.05 |
| | Compression ratio @800 cm/hr | 0.17 | 0.17 | 0.17 | 0.17 | 0.17 | 0.17 | 0.08 | 0.08 | 0.13 |
| | Dynamic binding capacity (DBC) mg/ml | 65 | 64 | 64 | 65 | 65 | 63 | 42 | 42 | - |

[Table 2]

| | | Example | | |
|---|---|---|---|---|
| | | 10 | 11 | 12 |
| Kind of carrier | Synthetic polymer-based affinity carrier | O | O | O |
| | Agarose-based affinity carrier | | | |
| | Ion exchange carrier | | | |
| Slurry liquid phase | Kind of salt | Citrate | Citrate | Citrate |
| | pKa of buffer (25°C) | pKa1=2.90<br>pKa2=4.35<br>pKa3=5.69 | pKa1=2.90<br>pKa2=4.35<br>pKa3=5.69 | pKa1=2.90<br>pKa2=4.35<br>pKa3=5.69 |
| | (pKa of buffer) - (Isoelectric point of carrier) | -0.65<br>(pKa2) | -0.65<br>(pKa2) | -0.65<br>(pKa2) |
| | Slurry liquid phase pH (25°C) | 5.0 | 5.0 | 5.0 |
| | (Slurry liquid phase pH) - (Isoelectric point of carrier) | ±0 | ±0 | ±0 |
| | Liquid composition | Water + EtOH | Water + EtOH | Water + EtOH |
| Characteristics | Isoelectric point of carrier PI (25°C) | 5.0 | 5.0 | 5.0 |
| | pH after substitution (25°C) | 4.5 | 5.0 | 4.5 |
| | Electrical conductivity after substitution mS/m (25°C) | 5.3 | 142 | 8.6 |
| Evaluation | Liquid permeability (cm/h) @0.25 MPa | 830 | 850 | 830 |
| | Back pressure (MPa) @800 cm/hr | 0.22 | 0.21 | 0.22 |
| | Compression ratio @800 cm/hr | 0.17 | 0.17 | 0.17 |
| | Dynamic binding capacity (DBC) mg/ml | 65 | - | - |

[Table 3]

| | | Comparative Example | | |
|---|---|---|---|---|
| | | 1 | 2 | 3 |
| Kind of carrier | Synthetic polymer-based affinity carrier | O | O | |
| | Agarose-based affinity carrier | | | |
| | Ion exchange carrier | | | O |
| Slurry liquid phase | Kind of salt | Phosphate | Phosphate | Acetate |
| | pKa of buffer (25°C) | pKa1=1.83<br>pKa2=6.63<br>pKa3=11.46 | pKa1=1.83<br>pKa2=6.63<br>pKa3=11.46 | pKa=4.57 |
| | (pKa of buffer) - (Isoelectric point of carrier) | -3.17<br>+1.63<br>+6.46 | -3.17<br>+1.63<br>+6.46 | -4.43 |
| | Slurry liquid phase pH (25°C) | 6.5 | 7.5 | 5.0 |
| | (Slurry liquid phase pH) - (Isoelectric point of carrier) | +1.5 | +2.5 | -4.0 |

| | Liquid composition | Water + EtOH | Water + EtOH | Water + EtOH |
|---|---|---|---|---|
| Characteristics | Isoelectric point of carrier PI (25°C) | 5.0 | 5.0 | 9.0 |
| | pH after substitution (25°C) | 7.5 | 8.0 | 5.0 |
| | Electrical conductivity after substitution mS/m (25°C) | 61.2 | 59.3 | 9.1 |
| Evaluation | Liquid permeability (cm/h) @0.25 MPa | 600 | 550 | 600 |
| | Back pressure (MPa) @800 cm/hr | 0.4 | 0.6 | 0.45 |
| | Compression ratio @800 cm/hr | 0.19 | 0.18 | 0.18 |
| | Dynamic binding capacity (DBC) mg/ml | 65 | 64 | - |

## Claims

1. A method for filling a column with a chromatography carrier, comprising Step 1 and Step 2 below:

   (Step 1) a substitution step of substituting a slurry with an aqueous solvent not containing a buffer, wherein the slurry contains a target substance-capturing chromatography carrier, a buffer having an acid dissociation constant (pKa) within a range of ±1.0 of an isoelectric point of the carrier and an aqueous solvent, and has a pH of a liquid phase adjusted within a range of ±2.0 of the isoelectric point of the carrier; and
   (Step 2) a filling step of filling a column with the slurry subjected to solvent substitution in Step 1.

2. The method according to claim 1,
   wherein the pH of the liquid phase of the slurry subjected to solvent substitution in Step 1 is in a range of 3 or more and 10 or less.

3. The method according to claim 1 or 2,
   wherein an electrical conductivity of the liquid phase of the slurry subjected to solvent substitution in Step 1 is in a range of 0.006 mS/m or more and 200 mS/m or less.

4. The method according to any one of claims 1 to 3,
   wherein the carrier is an affinity chromatography carrier or an ion exchange chromatography carrier.

5. The method according to any one of claims 1 to 4,
   wherein the buffer is a buffer selected from a compound having a plurality of acidic groups in a molecule and a salt thereof.

6. The method according to any one of claims 1 to 5,
   wherein a content of the buffer is 0.1 mM or more in the liquid phase of the slurry used in Step 1.

7. A method for storing a slurry which is used by substituting with an aqueous solvent not containing a buffer before filling to a chromatography column,

   wherein the slurry contains a target substance-capturing chromatography carrier, a buffer having an acid dissociation constant (pKa) within a range of ±1.0 of an isoelectric point of the carrier, and an aqueous solvent, and a pH of a liquid phase of the slurry is in a range of ±2.0 of the isoelectric point of the carrier.

8. A slurry which is used by substituting with an aqueous solvent not containing a buffer before filling to a column, comprising:

   a target substance-capturing chromatography carrier;
   a buffer having an acid dissociation constant (pKa) within a range of ±1.0 of an isoelectric point of the carrier; and an aqueous solvent,
   wherein a pH of a liquid phase is in a range of ±2.0 of the isoelectric point of the carrier.

**9.** The slurry according to claim 8,
wherein the buffer is a buffer selected from a compound having a plurality of acidic groups in a molecule and a salt thereof.

**INTERNATIONAL SEARCH REPORT**

International application No. **PCT/JP2022/033911**

**A. CLASSIFICATION OF SUBJECT MATTER**

***B01J 20/281***(2006.01)i; ***B01J 20/291***(2006.01)i; ***G01N 30/50***(2006.01)i; ***G01N 30/56***(2006.01)i
FI: G01N30/56 A; B01J20/281 R; B01J20/291; G01N30/50; G01N30/56 Z

According to International Patent Classification (IPC) or to both national classification and IPC

**B. FIELDS SEARCHED**

Minimum documentation searched (classification system followed by classification symbols)

B01J20/281; B01J20/291; G01N30/50; G01N30/56

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2015-507181 A (UPM-KYMMENE CORPORATION) 05 March 2015 (2015-03-05) paragraph [0034] | 1-9 |
| A | JP 2012-168054 A (SEKISUI MEDICAL CO LTD) 06 September 2012 (2012-09-06) paragraphs [0068]-[0069] | 1-9 |
| A | JP 2019-163263 A (MEDIMMUNE LTD) 26 September 2019 (2019-09-26) paragraphs [0064]-[0065] | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 November 2022** | **22 November 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/033911**

| Patent document cited in search report | Publication date (day/month/year) | Patent family member(s) | Publication date (day/month/year) |
|---|---|---|---|
| JP 2015-507181 A | 05 March 2015 | WO 2013/093196 A1<br>p. 7, lines 22-29<br>US 2014/0374254 A1<br>EP 2794051 A1 | |
| JP 2012-168054 A | 06 September 2012 | (Family: none) | |
| JP 2019-163263 A | 26 September 2019 | US 2016/0108084 A1<br>paragraphs [0091]-[0092]<br>EP 2997036 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020002028 A
- WO 2015199196 A
- WO 2017090658 A
- WO 2019039545 A
- JP 2011252929 A
- WO 2017155105 A
- JP 62025102 A
- WO 2015119255 A
- WO 2015041218 A
- US 6399750 B
- US 5260373 A
- JP 2010133733 A
- JP 2010133734 A
- JP 2011256176 A


### Non-patent literature cited in the description


- Chemistry Handbook. Maruzen Publishing
- Chemistry Handbook. Maruzen Publishing Co., Ltd,
- **LJUNGQUIST C. et al.** *rEur. J. Biochem.,* 1989, vol. 186, 557-561